# EUROPEAN PATENT SPECIFICATION

(11) **EP 4 637 875 B1**
(45) Date of publication and mention of the grant of the patent: **16.09.2026**
(21) Application number: 23833795.0
(22) Date of filing: 19.12.2023
(51) Int. Cl.: A61M 5/158, A61M 5/46, A61M 5/32, A61M 39/02

(54) **PORT CANNULA WITH BENDABLE NEEDLE**
PORTKANÜLE MIT BIEGBARER NADEL
CANULE D'ORIFICE À AIGUILLE PLIABLE

(30) Priority: 20.12.2022 EP 22215089
(43) Date of publication of application: 29.10.2025
(73) Proprietor: Fresenius Kabi Deutschland GmbH, 61352 Bad Homburg (DE)
(72) Inventor: DIMNI, Oltjon, 36251 Bad Hersfeld (DE); PÜTTER, Harry, 36364 Bad Salzschlirf (DE)
(74) Representative: Fresenius Kabi Deutschland GmbH
(86) International application number: PCT/EP2023/086523
(87) International publication number: WO 2024/133185

(56) References cited:
- EP-B1- 3 122 404
- WO-A1-2010/101573
- WO-A1-2013/090104
- WO-A1-2022/026035
- US-A1- 2013 331 786

## Description

The invention relates to a port cannula for puncturing a port and to a method for adjusting the port cannula.

Port cannulas are used for puncturing implanted port catheters. An implanted port catheter, in short also referred to as port, is arranged under the skin of a patient and typically defines a volume covered by a membrane. The volume of the port is connected to a catheter that opens into a vessel or other treatment site. To administer a liquid into the port, a port cannula with a needle is used. The needle is pressed through the skin of the patient and through the membrane into the volume so that the liquid can be filled into the volume of the port and, thereby, administered to the patient.

Such a port system is described in US 2013/0226103 A1.

Depending on weight and size of the patient, the thickness of the tissue covering the implanted port may differ strongly. Therefore, commonly a cannula with a correct needle length must be selected before the application. The needle length is selected to be just long enough to reach the volume of the port and at the same time allow a connecting piece of the port cannula to be in contact with the patient's skin to be secured thereto.

Due to the wide range of weights and sizes of the various patients, hospitals typically have to store a significant number of different needle lengths, e.g., six different lengths. Since regularly also different needle diameters may be applied, e.g., three different diameters, a large number of different port cannulas may have to be kept in a storeroom. In addition, the manufacturing of the port cannulas is complex, because the various different combinations of needle diameters and lengths need to be made.

Cannulas and needles with different measures to adjust a penetration depth are presented in the prior art. WO 2013/090104 A1 discloses a port cannula with a corresponding portal comprising a pivoting mechanism that enables the cannula to bend at the right length once it is inserted into an implanted port. Similarly, WO 2022/026035 A1 describes an assembly comprising a needle and a housing, securable to a skin of a patient, once the needle has punctured a port, actuation of an actuator roller impart a curvature along the needle to adjust its length. All of these are complex in manufacturing and handling.

It is an object of the instant invention to provide an improved port cannula.

This object is achieved by means of a port cannula comprising the features of claim 1.

Accordingly, a port cannula for puncturing a port comprises a base and a needle extending through the base. Therein, a tip of the needle is arranged at a first side of the base. It is provided that the needle is displaceable relative to the base to adjust a distance of the needle tip to the base, wherein the base has a support at (e.g., against) which the needle is bendable at a second side of the base opposite the first side.

This is based on the concept to replace a large number of cannulas with different needle lengths by just one type of an adjustable port cannula (per needle diameter). The length of the needle is easily adjustable by displacing the needle, and bending the needle at the support allows to avoid an unintended adjustment while puncturing an implanted port. In addition, bending the needle at the side of the base facing away the skin during puncturing allows an easy handling and a comfortable fit during infusion. Thus, an improved port cannula is provided. A port system may be provided that comprises the port cannula and the port.

The port cannula further comprises a removable protective cap accommodating a portion of the needle including the needle tip in an initial state. The protective cap may be removably mounted on the base. This allows to protect the needle before use. The needle may be displaceable with respect to the protective cap inside the protective cap.

The protective cap is at least partially transparent. For example, the protective cap may be made of a plastics material and/or a translucent material. This allows to inspect the needle, particularly the needle tip, inside the protective cap, particularly during adjustment.

A scale is provided on the protective cap. The scale indicates the distance of the needle tip to the base. The scale allows an easy adjustment of the correct distance. The scale may indicate the current needle length in centimeters and/or in inches. For example, the needle tip visible through transparent material of the protective cap indicates the distance on the scale. This allows a particularly simple and robust measurement of the length.

The base may comprise an attachment element for attachment to a patient's skin. Alternatively, or in addition, the base may comprise a locking element movable relative to the attachment element to lock the needle relative to the base. This allows to avoid an unintended displacement of the needle relative to the base while bending the needle. Thus, an accidental modification of the needle length before or during the bending can be avoided. Further, during puncturing, the needle is exposed and can be seen and inspected by the user. This simplifies puncturing by avoiding that the puncturing has to be done without actually seeing the needle. The base may be configured such that when operating the locking element, the needle is fixed to the base with a frictional connection.

The locking element may be rotatable relative to the attachment element. For example, the locking element is screwed on the attachment element. This allows to lock the needle in a simple manner. Alternatively, the locking element may comprise a button, a pin or the like.

For example, by rotation of the locking element relative to the attachment element, a pressure is exerted on the needle by the base. Particularly for this purpose, the attachment element and/or the locking element may have a conical shape. For example, pushing and/or screwing the locking element onto the attachment element may squeeze a part of the attachment element against the needle. This allows to lock the needle in a reliable manner with a simple construction.

As an example, the support of the base is formed on the locking element. By this, the locking element provides a dual functionality. Alternatively, the attachment element may provide the support (also providing a plurality of functions). The support may have a bending contour, wherein, after bending, the needle follows this bending contour.

According to an example, the support of the base describes a curvature. Optionally, the curvature has a curvature radius of 0.5 to 10 times the diameter of the needle, more particular, of 1 to 5 times the diameter of the needle. This allows a particularly controlled bending that avoids creating a kink in the needle even when bending the needle quickly.

A socket for connecting a tube (and, optionally, in connection with the tube) may be mounted on an end of the needle opposite the needle tip. Such a socket allows a simple and reliable connection, e.g., to a medication container providing a liquid to be infused. Optionally, the socket surrounds at least a part of the base in an initial state.

A part of the needle can be configured to be exposed between the base and the socket. This allows a very simple design that avoids bulky parts such as an additional housing.

Optionally, the needle comprises a form-lock element. The form-lock element may be configured to interact with a stop portion of the base, e.g., when the needle is retracted into the base. For example, the distal end of the needle has a curved form which can serve as the form-lock element. This allows to retract the needle tip into the base after use of the port cannula to avoid needlestick injuries in a secure and simple manner.

According to an aspect of the invention, a method for adjusting the port cannula according to any embodiment described herein is provided. The method comprises the following steps: displacing the needle relative to the base to adjust a needle length between the needle tip and the base at the first side of the base; and bending the needle at (e.g., against) the support at the second side of the base. The method may also comprise providing a predetermined distance of the needle tip to the base, wherein in the step of displacing the needle, a needle length in accordance with the predetermined distance is adjusted.

For the effects and advantages, reference is made to the description of the port cannula above. The method may further comprise, between the step of displacing the needle and the step of bending the needle, locking the needle relative to the base against further displacement, e.g., with the locking element described above.

A protective cap may accommodate a portion of the needle including the needle tip. The method may further comprise, after the step of bending the needle, removing the protective cap. Thus, the needle tip is protected during the step of bending the needle.

The idea underlying the invention shall subsequently be described in more detail by referring to the embodiments shown in the figures. Herein:
- Fig. 1: shows an implanted port catheter;
- Fig. 2: shows an adjustable port cannula for puncturing the port catheter according to Fig. 1, wherein the port cannula comprises a needle mounted so as to be bendable;
- Fig. 3: shows the port cannula according to Fig. 2, wherein a needle length is adjusted;
- Fig. 4: shows the port cannula according to Fig. 3, wherein the needle is bent;
- Fig. 5: shows the port cannula according to Fig. 4, wherein a protective cap is removed;
- Fig. 6: shows the port cannula according to Fig. 5 in a state puncturing the port of Fig. 1;
- Fig. 7: shows the port cannula according to Fig. 6 after removal from the port;
- Fig. 8: shows a support of a base of the port cannula having a curvature, as well as a possible needlestick protection mechanism;
- Fig. 9: shows a support of the base formed on a locking element and another possible needlestick protection mechanism; and

Figs. 10A and 10B show optional protection elements for the needle.

Subsequently, port cannulas for puncturing a port shall be described. The embodiments described herein shall not be construed as limiting for the scope of the invention.

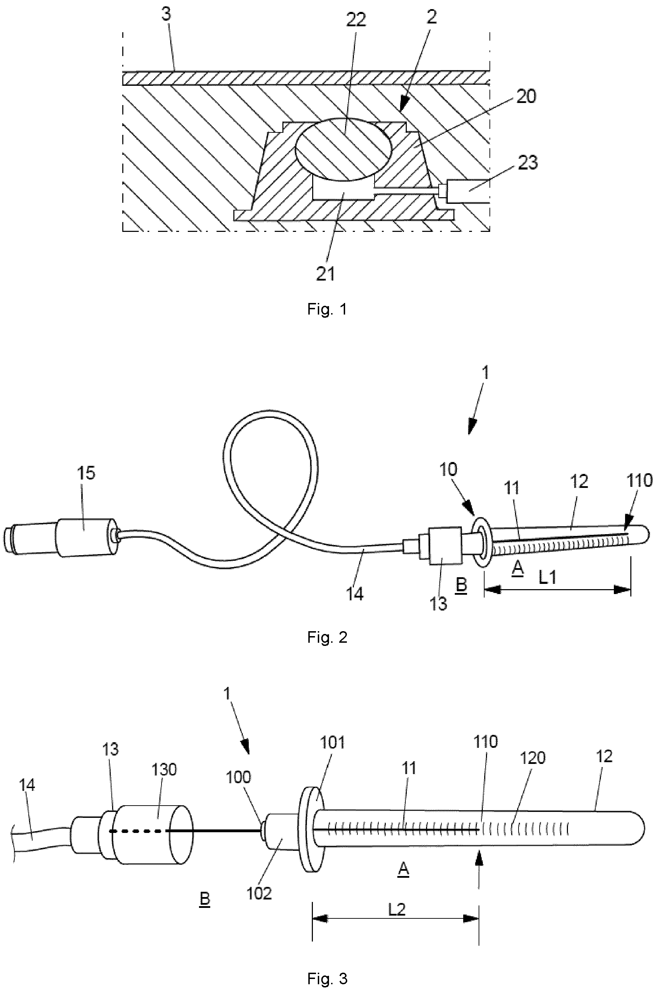

Fig. 1 shows an implantable port catheter, in the following referred to as port 2. In Fig. 1, the port 2 is shown in a state implanted under the skin 3 of a person. The port 2 comprises a housing 20 which defines an interior volume 21. A membrane 22 of the port 2 closes the volume 21. The volume 21 may also be referred to as infusion chamber. A catheter 23 is attached to the housing 20 and in fluid connection with the volume 21. The catheter 23 establishes a connection of the port 2 to a tissue, for example a vein. In order to give a patient a medicament that is to be administered intravenously, for example, the membrane 22 is pierced via the skin 3 by means of a port cannula. The port cannula establishes the connection between the infusion chamber volume 21 formed in the housing 20, e.g., to an infusion bag. As an example, a port catheter like the port 2 shown in Fig. 1 can have an implantation time of up to 5 years. Accordingly, the port 2 may be punctured often by a port cannula. The membrane 22 is made of a suitable material, e.g., silicone.

Figs. 2-7 show an adjustable port cannula 1 for puncturing the port 2 of Fig. 1. Together, the port cannula 1 and the port 2 form a port system.

The port cannula 1 comprises a base 10 and a needle 11 with a tip 110 at its end. The tip 110 is sharpened. The tip 110 has an inclined end surface. This allows to smoothly pierce through the membrane 22.

Further, the port cannula 1 comprises a protective cap 12. The protective cap 12 encloses the needle 11 in an initial state before application of the port cannula 1. Thus, the protective cap 12 protects the needle 11 tip 110 to avoid injuries.

A socket 13 of the port cannula 1 connects the needle 11 to one end of a tube 14. In the socket 13, the needle 11 and the tube 14 are in fluid connection. The end of the needle 11 opposite the tip 110 is arranged in the socket 13. The other end of the tube 14 is fixed to a connector 15. For example, the connector 15 is a needle-free connector. By means of the connector 15, the port cannula 1 can be provided with a liquid to be administered to a patient.

As can be seen, e.g., in Fig. 2, the needle 11 extends through the base 10. The tip 110 of the needle 11 is arranged at a first side A of the base 10. At a second side B, opposite the first side A, the needle is connected to the socket 13.

The needle 11 is slidably displaceable relative to the base 10 to adjust a distance L1 of the needle 11 tip 110 to the base 10. That is, the length (which can also be referred to as needle length) of the part of the needle 11 projecting from the base 10 on the first side A thereof, is adjustable.

Starting from Fig. 2 and with additional reference to Figs. 3 to 7, further details of the port cannula 1 and of a method of its adjustment will be described.

In the initial position, a cylindrical part 130 of the socket 13 may house the base 10 or a part thereof.

At first, a desired needle length is determined, e.g., based on the size and weight of a patient, or a measurement of the patient's skin. Then, this needle length is adjusted by displacing the needle 11 with respect to the base 10, e.g., holding the base 10 with one hand and pulling on the socket 13 with the other hand. To identify the correct distance L2 of the needle 11 tip 110 to the base 10, the protective cap 12 comprises a scale 120. Further, the protective cap 12 is made of a material that is transparent enough to allow the needle 11 inside the protective cap 12 to be seen through the material of the protective cap 12. Thus, starting from the initial state, the needle 11 is pulled so far until the correct distance L2, which equals or corresponds to the desired needle length, is indicated by the needle 11 tip 110 on the scale 120, see Fig. 3.

Notably, the base 10 comprises an attachment element 101 for attachment to the patient's skin 3, a support 100 and a locking element 102. The attachment element 101 comprises a flange for attachment, e.g., using tapes or the like, to the skin 3.

The locking element 102 can be moved from an unlocking position in which the needle 11 can be moved with respect to the base 10, to a locking position in which the needle 11 is fixed relative to the base 10.

After adjusting the desired needle length, the locking element 102 is moved from the unlocking position into the locking position. While this is not strictly necessary, adjustment can be simplified strongly thereby, because by locking the needle 11, an unintended further displacement of the needle 11 with respect to the base 10 can be avoided.

The support 100 supports the needle 11 at the second side B of the base 10. After pulling the needle 11 from the initial position to an adjusted position, e.g., as shown in Fig. 3, the needle 11 is exposed between the socket 13 and the base 10. At the support 100 and on the second side B the needle 11 is bendable. The base 10 allows the needle 11 to be bent.

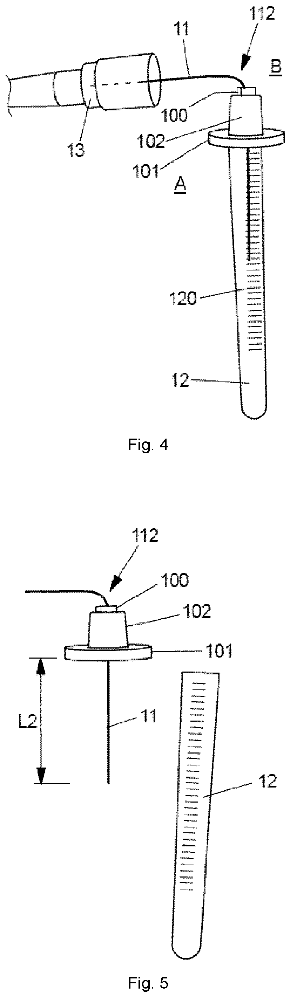

Hence, as illustrated in Fig. 4, after locking the needle 11 to the base 10, the needle 11 is bent. For example, the base 10 is held in one hand and the other hand is used to push the needle 11 to the side, against the support 100. The needle 11 is bendable with an angle of 90 degrees. As shown in Fig. 4, the needle 11 is bent by approximately 90 degrees. Before bending the needle 11, the needle 11 extends along a straight line. After bending, the needle 11 comprises a bent portion 112.

While bending the needle 11, the protective cap 12 can be maintained on the needle 11 to safely avoid injuries while bending.

As shown in Fig. 5, after bending the needle 11, the protective cap 12 can be removed from the needle 11 and base 10. The protective cap 12 may be mounted on the base by a snap fit, by a screw thread, by a bayonet catch or the like.

Then, the needle 11 tip 110 is exposed and at the adjusted distance L2 to the base 10 (more specifically, to the surface of the base 10, namely, of the attachment element 101, closest to the needle 11 tip 110).

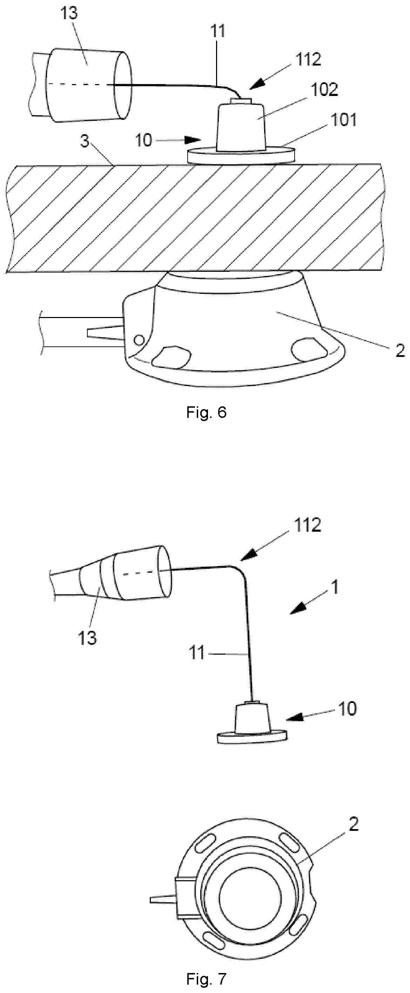

Next, as shown in Fig. 6, the needle can be punctured through the skin 3 and through the membrane 22 of the port 2 into the inner volume 21 of the port 2. A fluid can then be administered. As can be seen in Fig. 6, due to the bent needle 11, a height of the port cannula above the skin 3 is comparably low. This allows a comfortable and ergonomic fit. The unused length of the needle 11 is bent (and stowed) aside.

When the infusion is completed, the port cannula 1 can be removed from the patient. This can be done (by hand or with a tool) in an easy manner by unlocking the locking element 102 and then pulling the needle 11 out of the port 2 and skin 3 while maintaining, e.g., holding the base 10 on the skin 3. Thereby, the needle 11 is displaced relative to the base 10, until the needle 11 tip 110 is arranged inside the base 10.

This is shown in Fig. 7. The needle 11 tip 110 is secured within the base 10 against accidental needlestick injuries.

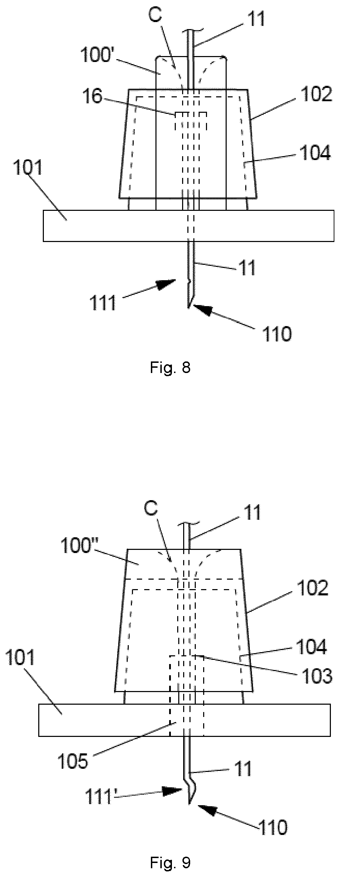

Fig. 8 shows several features which the base 10 and the needle 11 of the port cannula 1 of Figs. 2-7 may have.

First, the needle 11 may have one or more form-lock elements 111. According to Fig. 8, the form-lock element 111 is formed as a depression in the needle 11, more specifically, as a groove in the needle 11. Here, the form-lock element 111 is made by stamping the needle 11. The form-lock element 111 is arranged adjacent the needle 11 tip 110. So, the form-lock element 11 is made in one piece with the remainder of the needle 11. The base 10 may be designed such that the needle 11 including the form-lock element 111 may be pulled through the base 10. The base 10 has a through hole through which the needle 11 extends, and this through hole may be large enough to allow the needle 11 including the form-lock element 111 to pass through. In case that the needle 11 alternatively, or in addition, comprises a form-lock element 111' as described below with reference to Figs. 9-10B for interacting with the protection element 16, the through hole may be likewise large enough to let the needle 11 including the form-lock element 111' pass through.

As an option, the port cannula 1 may comprise a protection element 16. The protection element 16 may be formed as a cage or the like. The protection element 16 is arranged on or in the base 10. When pulling the needle 11 through the base 10, the form-lock element 111 is engaged by the protection element 16, e.g., in the manner as in common vein cannulas, thereby covering the needle 11 tip 110 with the protection element 16. To this end, the protection element 16 is removably accommodated in (or on) the base 10.

The locking element 102 is rotatably mounted on the attachment element 101. As shown in Fig. 8, the attachment element 101 has a portion onto which the locking element 102 is screwed. This portion of the attachment element 101 is conical and, therefore, referred to as conical portion 104. Also, the locking element 102 is conical. The conical portion 104 has an external thread. The locking element 102 has an internal thread. When screwing the locking element 102 on the conical portion 104, the conical portion 104 is compressed and, thereby, compresses the needle 11 therein. By this, the needle 11 is fixed relative to the base 10.

Further, according to Fig. 8, a support 100', against which the needle 11 can be bent, is formed by the attachment element 101 (or an element fixed thereto). The support 100' describes a curvature C with a curvature radius of 0.5 to 10 times the diameter of the needle 11, more specifically, of 1 to 5 times the diameter of the needle 11.

Fig. 9 shows further features which the base 10 and the needle 11 of the port cannula 1 of Figs. 2-7 may have.

According to Fig. 9, a support 100" against which the needle 11 can be bent, is formed by the locking element 102. Therefore, the locking element 102 has a curvature C, against which the needle 11 may be bent so as to follow the curvature C. The curvature C may have a curvature radius as described above.

The base 10 forms a receptacle 105 on its first side A for a form-lock element 111' of the needle 11. Hence, when retracting the needle 11 tip 110 into the base 10, the form-lock element 111' and the tip 110 enter the receptacle 105 until the form-lock element 111' interacts with a stop portion 103 of the base 10. In the present example, the form-lock element 111' is formed by a curved shape of a distal end portion of the needle 11 having the tip 110. The curved shape does not fit into the through hole of the base 10 in the example of Fig. 9.

This prevents the needle 11 to be accidentally pulled entirely through the base 11, separating the needle 11 from the base 10 and exposing the tip 110 again.

Notably, the base 10 can fulfil a number of functions. It adjustably holds the needle 11. It provides a locking mechanism for the needle 11. It serves as a housing for a needlestick-protection mechanism. It retains the protective cap 12. It comprises a plate or flange to be secured to the skin 3 of the patient in a comfortable manner.

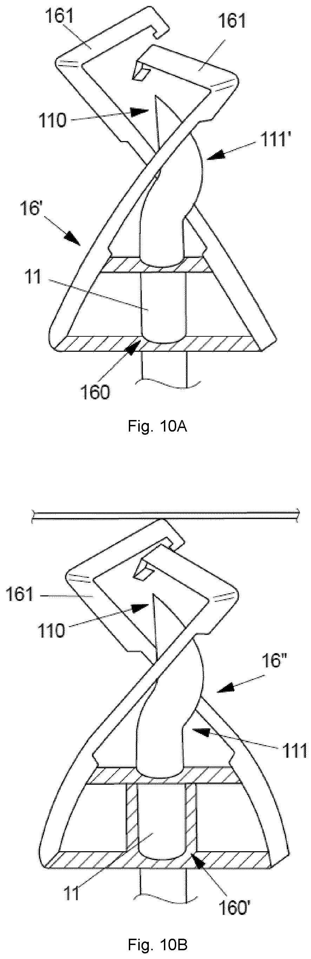

Figs. 10A and 10B show two possible protection elements 16', 16" for the port cannula 1. Each of the protection elements 16', 16" may be mounted on or in the base 10 as described above with reference to Fig. 8.

In the present examples, each of the protection elements 16', 16" is made of metal, such as a spring steel. Here, the respective protection elements 16', 16" are made in one piece. Further, the protection elements 16', 16" are made of (flat) sheet material which is bent several times. Each of the protection elements 16', 16" is formed as a needle safety clip. Further, each of the protection elements 16', 16" comprises at least one clamp element 161, here two clamp elements 161, and a needle guide 160, 160'. By means of the needle guide 160, 160', the respective protection element 16', 16" is slidable along the needle 11. However, the needle guide 160, 160' prevents the form-lock element 111' (in this example the curved portion of the needle 11) to pass through the needle guide 160, 160'. The needle guide 160, 160' also prevents a tilting of the protection element 16', 16" with respect to the needle 11.

When pulling the needle 11 out of the base 10, the curved portion of the needle 11 interacts with the needle guide 160, 160' so that the respective protection element 16', 16" is pulled off from the base 10 together with the needle 11. As such, the respective protection element 16', 16" houses the needle 11 tip 110. The clamp elements 161 project over the needle 11 tip 110 and, thereby, can prevent needlestick injuries. The clamp elements 161 cross each other.

The needle guide 160 of the protection element 16' of Fig. 10A comprises two guiding sections that each are in contact with the needle 11 and are spaced apart from one another. Between the two guiding sections the needle 11 is exposed.

The needle guide 160' of the protection element 16" of Fig. 10A comprises one guiding section forming a cylindrical area of contact with the needle 11 wherein, in the present example, the cylinder length is larger than the cylinder diameter.

The idea of the invention is not limited to the embodiments described above but may be implemented in a different fashion. The invention is defined by the appended claims.

### List of Reference Numerals

- 1: Port cannula
- 10: Base
- 100; 100'; 100": Support
- 101: Attachment element
- 102: Locking element
- 103: Stop portion
- 104: Conical portion
- 105: Receptacle
- 11: Needle
- 110: Tip
- 111; 111': Form-lock element
- 112: Bent portion
- 12: Protective cap
- 120: Scale
- 13: Socket
- 130: Cylindrical part
- 14: Tube
- 15: Connector
- 16; 16'; 16": Protection element
- 160; 160': Needle guide
- 161: Clamp element
- 2: Port
- 20: Housing
- 21: Volume
- 22: Membrane
- 23: Catheter
- 3: Skin
- A: First side
- B: Second side
- C: Curvature
- L1, L2: Distance

## Claims

1. A port cannula (1) for puncturing a port (2), the port cannula (1) comprising:
- a base (10) and
- a needle (11) extending through the base (10), wherein a tip (110) of the needle (11) is arranged at a first side (A) of the base (10),
**wherein**
the needle (11) is displaceable relative to the base (10) to adjust a distance (L1, L2) of the needle (11) tip (110) to the base (10), wherein the base (10) has a support (100; 100'; 100") at which the needle (11) is bendable at a second side (B) of the base (10) opposite the first side (A),
**characterized in** further comprising
- a removable protective cap (12) accommodating a portion of the needle (11) including the needle (11) tip (110), wherein the protective cap (12) is at least partially transparent and wherein a scale (120) is provided on the protective cap (12) indicating the distance (L1, L2) of the needle (11) tip (110) to the base (10).

2. The port cannula (1) according to any of the preceding claims, **characterized in that** the base (10) comprises an attachment element (101) for attachment to a patient's skin (3) and a locking element (102) movable relative to the attachment element (101) to lock the needle (11) relative to the base (10).

3. The port cannula (1) according to claim 2, **characterized in that** the locking element (102) is rotatable relative to the attachment element (101).

4. The port cannula (1) according to claim 3, **characterized in that** by rotation of the locking element (102) relative to the attachment element (101) a pressure is exerted on the needle (11) by the base (10).

5. The port cannula (1) according to any of claims 2 to 4, **characterized in that** the support (100") of the base (10) is formed on the locking element (102).

6. The port cannula (1) according to any of the preceding claims, **characterized in that** the support (100'; 100") of the base (10) describes a curvature (C) with a curvature radius of 0.5 to 10 times the diameter of the needle (11).

7. The port cannula (1) according to any of the preceding claims, **characterized in that** a socket (13) for connecting a tube (14) is mounted on an end of the needle (11) opposite the needle (11) tip (110).

8. The port cannula (1) according to claim 7, **characterized in that** a part of the needle (11) is configured to be exposed between the base (10) and the socket (13).

9. The port cannula (1) according to any of the preceding claims, **characterized in that** the needle (11) comprises a form-lock element (111; 111') that interacts with a stop portion (103) of the base (10) when the needle (11) is retracted into the base (10).

10. A method for adjusting the port cannula (1) according to any of the preceding claims, **characterized by:**
- displacing the needle (11) relative to the base (10) to adjust a needle length between the needle (11) tip (110) and the base (10) at the first side (A) of the base (10); and
- bending the needle (11) at the support (100; 100'; 100") at the second side (B) of the base (10).

11. The method according to claim 10, **characterized in that** the method further comprises, between the step of displacing the needle (11) and the step of bending the needle (11):
- locking the needle (11) relative to the base (10) against further displacement.

12. The method according to claim 10 or 11, **characterized in that** the method further comprises, after the step of bending the needle (11):
- removing the protective cap (12).

## Patentansprüche

1. Portkanüle (1) zum Durchstechen eines Ports (2), wobei die Portkanüle (1) Folgendes umfasst:
- eine Basis (10) und
- eine Nadel (11), die sich durch die Basis (10) hindurch erstreckt, wobei eine Spitze (110) der Nadel (11) an einer ersten Seite (A) der Basis (10) angeordnet ist,
**wobei**
die Nadel (11) relativ zur Basis (10) verschiebbar ist, um einen Abstand (L1, L2) der Spitze (110) der Nadel (11) zur Basis (10) einzustellen, wobei die Basis (10) einen Halter (100; 100. 100") aufweist, an dem die Nadel (11) an einer der ersten Seite (A) gegenüberliegenden zweiten Seite (B) der Basis (10) biegbar ist,
**dadurch gekennzeichnet, dass** sie ferner Folgendes umfasst
- eine abnehmbare Schutzkappe (12), die einen Abschnitt der Nadel (11) einschließlich der Spitze (110) der Nadel (11) aufnimmt, wobei die Schutzkappe (12) zumindest teilweise transparent ist und wobei an der Schutzkappe (12) eine Skala (120) vorgesehen ist, die den Abstand (L1, L2) der Spitze (110) der Nadel (11) zur Basis (10) angibt.

2. Portkanüle (1) nach einem der vorhergehenden Ansprüche, **dadurch gekennzeichnet, dass** die Basis (10) ein Befestigungselement (101) zur Befestigung an der Haut (3) eines Patienten und ein Verriegelungselement (102) umfasst, das relativ zum Befestigungselement (101) bewegbar ist, um die Nadel (11) relativ zur Basis (10) zu verriegeln.

3. Portkanüle (1) nach Anspruch 2, **dadurch gekennzeichnet, dass** das Verriegelungselement (102) relativ zum Befestigungselement (101) drehbar ist.

4. Portkanüle (1) nach Anspruch 3, **dadurch gekennzeichnet, dass** durch Drehen des Verriegelungselements (102) relativ zum Befestigungselement (101) ein Druck auf die Nadel (11) durch die Basis (10) ausgeübt wird.

5. Portkanüle (1) nach **einem** der Ansprüche 2 bis 4, **dadurch gekennzeichnet, dass** der Halter (100") der Basis (10) am Verriegelungselement (102) ausgebildet ist.

6. Portkanüle (1) nach einem der vorhergehenden Ansprüche, **dadurch gekennzeichnet, dass** der Halter (100'; 100") der Basis (10) eine Krümmung (C) mit einem Krümmungsradius vom 0,5- bis 10-fachen des Durchmessers der Nadel (11) beschreibt.

7. Portkanüle (1) nach **einem** der vorhergehenden Ansprüche, **dadurch gekennzeichnet, dass** an einem der Spitze (110) der Nadel (11) gegenüberliegenden Ende der Nadel (11) eine Buchse (13) zum Anschließen eines Schlauchs (14) angebracht ist.

8. Portkanüle (1) nach Anspruch 7, **dadurch gekennzeichnet, dass** ein Teil der Nadel (11) derart konfiguriert ist, dass er zwischen der Basis (10) und der Buchse (13) freiliegt.

9. Portkanüle (1) nach einem der vorhergehenden Ansprüche, **dadurch gekennzeichnet, dass** die Nadel (11) ein Formschlusselement (111; 111') umfasst, das mit einem Anschlagabschnitt (103) der Basis (10) zusammenwirkt, wenn die Nadel (11) in die Basis (10) eingezogen wird.

10. Verfahren zum Einstellen der Portkanüle (1) nach einem der vorhergehenden Ansprüche, **gekennzeichnet durch:**
- Verschieben der Nadel (11) relativ zur Basis (10), um eine Nadellänge zwischen der Spitze (110) der Nadel (11) und der Basis (10) an der ersten Seite (A) der Basis (10) einzustellen; und
- Biegen der Nadel (11) an dem Halter (100; 100'; 100") an der zweiten Seite (B) der Basis (10).

11. Verfahren nach Anspruch 10, **dadurch gekennzeichnet, dass** das Verfahren ferner zwischen dem Schritt des Verschiebens der Nadel (11) und dem Schritt des Biegens der Nadel (11) Folgendes umfasst:
- Verriegeln der Nadel (11) relativ zur Basis (10) gegen weitere Verschiebung.

12. Verfahren nach Anspruch 10 oder 11, **dadurch gekennzeichnet, dass** das Verfahren nach dem Schritt des Biegens der Nadel (11) ferner Folgendes umfasst:
- Entfernen der Schutzkappe (12).

## Revendications

1. Canule à orifice (1) pour la perforation d'un orifice (2), la canule à orifice (1) comprenant :
- une base (10) et
- une aiguille (11) s'étendant à travers la base (10), dans laquelle une pointe (110) de l'aiguille (11) est agencée au niveau d'un premier côté (A) de la base (10),
**dans laquelle**
l'aiguille (11) est déplaçable par rapport à la base (10) pour ajuster une distance (L1, L2) de la pointe (110) de l'aiguille (11) jusqu'à la base (10), dans laquelle la base (10) a un support (100 ; 100' ; 100") au niveau duquel l'aiguille (11) peut être pliée au niveau d'un second côté (B) de la base (10) opposé au premier côté (A),
**caractérisée en ce qu'**elle comprend en outre
- un capuchon de protection retirable (12) logeant une partie de l'aiguille (11) comportant la pointe (110) de l'aiguille (11), dans laquelle le capuchon de protection (12) est au moins partiellement transparent et dans laquelle une échelle (120) est fournie sur le capuchon de protection (12) indiquant la distance (L1, L2) de la pointe (110) de l'aiguille (11) jusqu'à la base (10).

2. Canule à orifice (1) selon l'une quelconque des revendications précédentes, **caractérisée en ce que** la base (10) comprend un élément de fixation (101) pour une fixation à la peau d'un patient (3) et un élément de verrouillage (102) mobile par rapport à l'élément de fixation (101) pour verrouiller l'aiguille (11) par rapport à la base (10).

3. Canule à orifice (1) selon la revendication 2, **caractérisée en ce que** l'élément de verrouillage (102) peut être entraîné en rotation par rapport à l'élément de fixation (101).

4. Canule à orifice (1) selon la revendication 3, **caractérisée en ce que** par rotation de l'élément de verrouillage (102) par rapport à l'élément de fixation (101), une pression est exercée sur l'aiguille (11) par la base (10).

5. Canule à orifice (1) selon l'**une quelconque** des revendications 2 à 4, **caractérisée en ce que** le support (100") de la base (10) est formé sur l'élément de verrouillage (102).

6. Canule à orifice (1) selon l'une quelconque des revendications précédentes, **caractérisée en ce que** le support (100' ; 100") de la base (10) décrit une courbure (C) avec un rayon de courbure de 0,5 à 10 fois le diamètre de l'aiguille (11).

7. Canule à orifice (1) selon l'**une quelconque** des revendications précédentes, **caractérisée en ce qu'une** douille (13) pour relier un tube (14) est montée sur une extrémité de l'aiguille (11) opposée à la pointe (110) de l'aiguille (11).

8. Canule à orifice (1) selon la revendication 7, **caractérisée en ce qu'une** partie de l'aiguille (11) est conçue pour être exposée entre la base (10) et la douille (13).

9. Canule à orifice (1) selon l'**une quelconque** des revendications précédentes, **caractérisée en ce que** l'aiguille (11) comprend un élément de verrouillage de forme (111 ; 111') qui interagit avec une partie de butée (103) de la base (10) lorsque l'aiguille (11) est rétractée dans la base (10).

10. Procédé d'ajustement de la canule à orifice (1) selon l'une quelconque des revendications précédentes, **caractérisé par :**
- le déplacement de l'aiguille (11) par rapport à la base (10) pour ajuster une longueur d'aiguille entre la pointe (110) de l'aiguille (11) et la base (10) au niveau du premier côté (A) de la base (10) ; et
- le pliage de l'aiguille (11) au niveau du support (100 ; 100' ; 100") au niveau du second côté (B) de la base (10).

11. Procédé selon la revendication 10, **caractérisé en ce que** le procédé comprend en outre, entre l'étape de déplacement de l'aiguille (11) et l'étape de pliage de l'aiguille (11) :
- le verrouillage de l'aiguille (11) par rapport à la base (10) contre tout déplacement supplémentaire.

12. Procédé selon la revendication 10 ou 11, **caractérisé en ce que** le procédé comprend en outre, après l'étape de pliage de l'aiguille (11) :
- le retrait du capuchon de protection (12).
